# EUROPEAN PATENT SPECIFICATION

(11) **EP 0 715 840 B1**
(45) Date of publication and mention of the grant of the patent: **14.06.2000**
(21) Application number: 95308727.7
(22) Date of filing: 04.12.1995
(51) Int. Cl.: A61F 13/15

(54) **Disposable diaper**
Wegwerfwindel
Couche-culotte jettable

(30) Priority: 05.12.1994 JP 30103694
(43) Date of publication of application: 12.06.1996
(73) Proprietor: UNI-CHARM CORPORATION, Kawanoe-shi Ehime-ken (JP)
(72) Inventor: Takamitsu, Igaue, Kawanoe-shi, Ehime-ken (JP); Kouji Inoue, Kannonji-shi, Kagawa-ken (JP); Kido, Tsutomu, Kawanoe-shi, Ehime-ken (JP)
(74) Representative: Murgatroyd, Susan Elizabeth

(56) References cited:
- EP-A- 0 437 771
- US-A- 5 074 854

## Description

The present invention relates to disposable diaper and, more particularly to a disposable diaper for the absorption and containment of urine and other bodily exudates.

Conventional disposable diapers of open type or pants type are provided with elastic members with a tension substantially along the entire circumferences of the waist-hole and leg-holes in order to improve its fitting to the infant's body and a preventive effect against bodily exudates leakage. These elastic members contract to form gathers around the waist-hole and around the leg-holes.

However, the known diapers have been inconvenient for use in that, particularly when the open type diaper is put on with the infant lying face up, it is difficult to seize the waist side edges of the rear section of the diaper which tightly contract to form gathers and hide under the infant's back, and therefore to put the diaper on the infant rapidly. Also the pants type diaper has been inconvenient for use in that both its waist-hole and its leg-holes tightly contract to form gathers, making it difficult to rapidly distinguish the waste-hole from the leg-holes.

In view of the problems left behind by the prior art, it is a principal object of the invention to solve the problems by varying an elongation ratio of an elastic member attached to a waist portion of at least one of front and rear bodies of a diaper.

Accordingly, the present invention consists in a disposable diaper comprising a front section having a first waist portion; a rear section having a second waist portion; a crotch section having a pair of leg surrounding portions at lateral opposite sides thereof; and elastic means provided along the leg surrounding portions and at least one of the first and second waist portions, respectively, characterised in that the elastic means provided along the or each waist portion have an elongation ratio adjusted to be gradually decreased from a transversely middle zone to opposite side edges of the waist portion .

With the disposable diaper described above, gathers formed along the waist portion are concentrated in the middle zone and gradually decreased toward the transversely opposite sides of the waist portion.

The invention will now be described by way of example with reference to the accompanying drawings, in which:-
Fig. 1 is a perspective view showing a disposable diaper in accordance with one embodiment of the invention in its extended state as partially broken away; and
Fig. 2 is a perspective view showing the same in its unextended state.

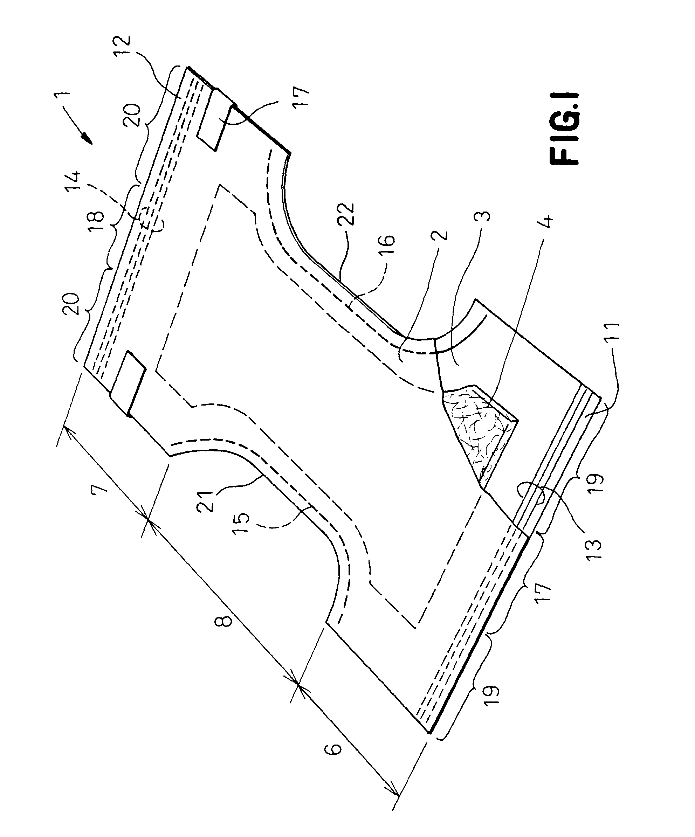

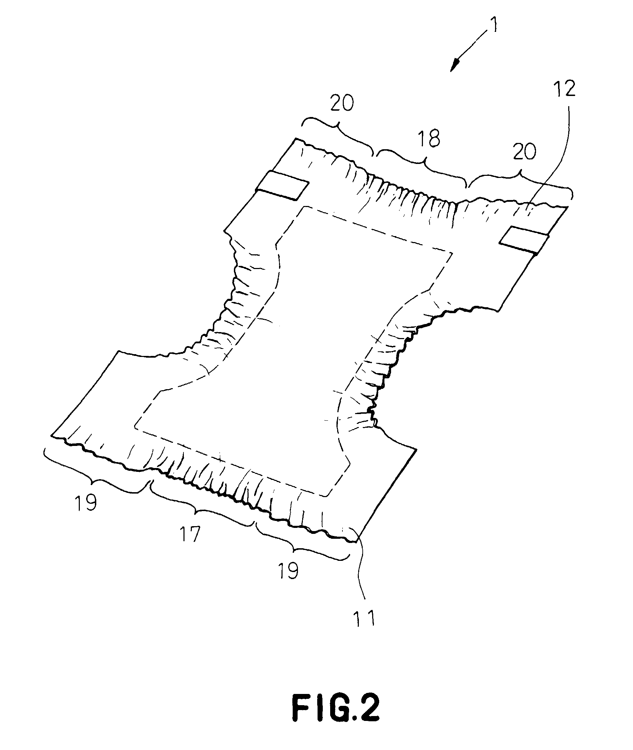

Referring to Figs. 1 and 2, a disposable diaper 1 of open type comprises a liquid-permeable topsheet 2, a liquid-impermeable backsheet 3 and a liquid-absorbent core 4 disposed between these two sheets 2,3, and longitudinally formed by a front section 6, a rear section 7 and a crotch section 8 interposed between these two sections 6,7. Waist portions 11, 12 of the front and rear sections 6, 7 are provided along their transverse direction with elastic members 13,14 and a pair of leg surrounding portions 21, 22 are provided along their surrounding direction with elastic members 15, 16. Well known tape fasteners 17 are attached to transversely opposite sides of the rear section 7.

Each of the elastic members 13, 14 for the waist portions 11, 12 comprises a desired number of elastic elements and each of them is bonded with a tension to an inner surface of the top- or backsheet 2, 3. One or both of the elastic members 13, 14 has an elongation ratio of 1.5 to 2.5 in a transversely middle zone 17 or 18 of the diaper 1 but, in zones 19, 20 on either side of the middle zone 17 or 18, the elongation ratio gradually decreases substantially to 1.0 closely adjacent its transversely opposite sides. The middle zone 17 or 18 may occupy 1/5 to 1/2 of the full length. When the elongation of one of the elastic members 13 or 14 is not adjusted to be higher in its middle zone 17 or 18, it is preferred to make the elongation ratio of the unadjusted elastic member 13 or 14 substantially uniform over its full length. It should be understood that, when the elongation ratio is made uniform, such arrangement should be made for the elastic member 13 on the front section in order to avoid an apprehension that the wearer's belly might be locally compressed.

The elastic members 15, 16 for the leg surrounding portions 21, 22 also comprise a desired number of elastic elements each bonded with a tension to the inner surface of the top- or backsheet 2, 3 substantially along the surrounding direction of the leg surrounding portions 21, 22.

When such diaper 1 is in its unextended state, the waist portions 11, 12 contract mainly in the middle zones 17, 18 to form a plurality of fine gathers but, in the zones 19, 20 on either side thereof, the gathers are formed very sparsely, as shown by Fig. 2. Around the leg surrounding portions 21, 22, on the other hand, gathers are formed uniformly. With the diaper 1 put against the hips of an infant lying face up, the side zones 19, 20 can be reliably as well as rapidly seized to put the diaper 1 on the infant's body, since these zones 19, 20 are free from significant contraction and maintained in a flat state. When the invention is applied to the pants type diaper, many gathers are formed in the transversely middle zone of the waist portions but, in the side edge zones, the formation of gathers is very sparse, so the waist portion can be easily distinguished from the leg surrounding portions having gathers along their surrounding direction.

In the diaper 1 of the invention, materials usually employed in the relevant technical field may be employed for the top- and backsheets 2, 3, the liquid-absorbent core 4 and the elastic members 13, 14, 15, 16. Bonding of the respective members may be achieved by using a suitable adhesive agent such as hot melt adhesive or by using a heat sealing technique.

With the inventive diaper, the formation of gathers can be concentrated in the transversely middle zone along the waist line and the zones on either side of this middle zone can be maintained in a substantially flat state by making the formation of gathers extremely sparse, because the elongation ratio of the elastic members extending along the waist lines of the front and rear sections is adjusted to the maximum in the transversely middle zone and to be gradually decreased toward the transversely opposite side edges of the diaper. In the case of open type diaper, the lateral zones can be reliably as well as rapidly seized so that the diaper may be easily put on the infant's body. In the case of pants type diaper, the waist portions can be readily distinguished from the leg surrounding portions. Thus, the pants type diaper also can be easily put on the infant's body.

## Claims

1. A disposable diaper comprising a front section (6) having a first waist portion (11); a rear section (7) having a second waist portion (12); a crotch section (8) having a pair of leg surrounding portions (21,22) at lateral opposite sides thereof; and elastic means (13,14,15,16) provided along the leg surrounding portions (21,22) and at least one of the first and second waist portions (11,12), respectively, characterised in that the elastic means (13,14) provided along the or each waist portion (11,12) have an elongation ratio adjusted to be gradually decreased from a transversely middle zone (17,18) to opposite side edges of the waist portion (11,12).

2. A disposable diaper according to claim 1, wherein said diaper comprises a liquid-permeable topsheet (2), a liquid-impermeable backsheet (3), and a liquid-absorbent core (4) disposed between these two sheets (2,3).

3. A disposable diaper according to claim 1 or 2, wherein the elongation ratio is adjusted to be approximately 1.5 to 2.5 in said middle zone (17,18) and to be approximately 1.0 closely adjacent to said opposite side edges of the waist portion (11,12).

## Patentansprüche

1. Wegwerfwindel, umfassend einen vorderen Abschnitt (6) mit einem ersten Hüftteil (11); einen hinteren Abschnitt (7) mit einem zweiten Hüftteil (12); einen Schritteil (8) mit einem Paar beinumgebender Bereiche (21, 22) an dessen seitlich sich gegenüberliegenden Seiten; und elastische Mittel (13, 14, 15, 16), die entlang der beinumgebenden Bereiche (21, 22) beziehungsweise wenigstens eines des ersten und des zweiten Hüftteils (11, 12) vorgesehen sind, dadurch gekennzeichnet, dass die elastischen Mittel (13, 14), die entlang des oder jedes Hüftteils (11, 12) vorgesehen sind, einen abgestimmten Verlängerungsgrad aufweisen, der von einer quer verlaufenden Mittelzone (17, 18) zu gegenüberliegenden Seitenkanten des Hüftteils (11, 12) allmählich abnimmt.

2. Wegwerfwindel gemäß Anspruch 1, wobei die Windel eine flüssigkeitsdurchlässige Oberlage (2), eine flüssigkeitsundurchlässige Unterlage (3) und einen flüssigkeitsabsorbierenden Kern (4) umfasst, der zwischen diesen beiden Lagen (2, 3) vorgesehen ist.

3. Wegwerfwindel gemäß Anspruch 1 oder 2, wobei der Verlängerungsgrad annähernd auf 1,5 bis 2,5 in der Mittelzone (17, 18) und auf annähernd 1,0 ziemlich nahe an den gegenüberliegenden Seitenkanten des Hüftteils (11, 12) eingestellt ist.

## Revendications

1. Couche-culotte jetable comprenant une section avant (6) ayant une première partie de ceinture (11); une section arrière (7) ayant une seconde partie de ceinture (12) ; une section de fourche (8) ayant une paire de parties entourant la jambe (21, 22) sur des côtés latéraux opposés de celle-ci ; et des moyens élastiques (13, 14, 15, 16) prévus le long des parties (21, 22) entourant les jambes et d'au moins une parmi les première et seconde parties de ceinture (11 ;, 12) respectivement, caractérisée en ce que les moyens élastiques (13, 14) prévus le long de la ou de chaque partie de ceinture (11, 12) ont un taux d'allongement ajusté de manière à décroître graduellement à partir d'une zone transversalement médiane (17, 18) vers les bords latéraux opposés de la partie de ceinture (11, 12).

2. Couche-culotte selon la revendication 1, caractérisée en ce que ladite couche-culotte comprend une feuille supérieure perméable au liquide (2), une feuille arrière imperméable au liquide (3), et un noyau (4) absorbant le liquide disposé entre ces deux feuilles (2, 3).

3. Couche-culotte selon la revendication 1 ou 2, caractérisée en ce que le taux d'allongement est ajusté pour être environ de 1,5 à 2,5 dans ladite zone médiane (17, 18) et pour être environ de 1,0 à proximité adjacente desdits bords latéraux opposés de la partie de ceinture (11, 12).
